# EUROPEAN PATENT APPLICATION

(11) **EP 4 265 629 A1**
(43) Date of publication of application: **25.10.2023**
(21) Application number: 21911090.5
(22) Date of filing: 24.12.2021
(51) Int. Cl.: C07K 1/02, C07K 5/062

(54) **METHOD FOR PRODUCING LIPID PEPTIDE**

(30) Priority: 25.12.2020 JP 2020217730
(71) Applicant: Nissan Chemical Corporation, Tokyo 103-6119 (JP)
(72) Inventor: SHOJI, Takeaki, Funabashi-shi, Chiba 274-0052 (JP)
(74) Representative: Hoffmann Eitle
(86) International application number: PCT/JP2021/048365
(87) International publication number: WO 2022/138953

(57) **Abstract**

A method for producing a lipid peptide compound represented by formula (3) or a pharmaceutically usable salt thereof, the method comprising: a reaction step of reacting an ester compound of the following Formula (1) with an α-amino acid compound of the following Formula (2) and a base in a solvent containing a non-polar organic solvent; an extraction step of adding an organic acid to a solution containing a salt of a lipidic peptide prepared through the reaction step and being of the following Formula (3) to thereby neutralize the solution, adding water and an alcohol to the neutralized solution, and subjecting the resultant mixture to phase separation, thereby removing the non-polar organic solvent; and a separation step of removing the lipidic peptide compound of Formula (3) from the solution prepared through the extraction step to the outside of the system.

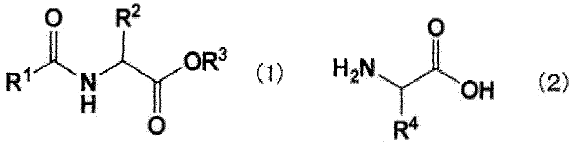

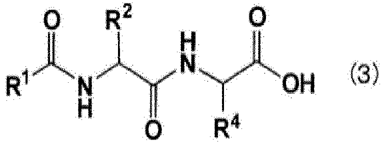

## Description

### TECHNICAL FIELD

The present invention relates to a method for producing a lipidic peptide.

### BACKGROUND ART

In recent years, a proposal has been made to use, as a hydrogelator, a novel lipidic peptide (lipidic peptide compound) prepared by binding of glycine or histidine to palmitic acid, etc., and a method of supplying the lipidic peptide has become important (Patent Document 1).

Meanwhile, a method involving solid-phase peptide synthesis has been generally shown as a production method for a lipidic peptide. However, the method can only be used for small-scale synthesis, and is difficult to apply to large-scale production.

Hitherto, the present inventors have reported that a lipidic peptide compound can be directly produced through reaction (amidation) between an amino group of an amino acid and an ester compound in the presence of a base in a solvent containing a non-polar organic solvent without use of a protective group (Patent Document 2).

### Prior Art Documents

### Patent Documents

Patent Document 1: WO 2010/013555
Patent Document 2: WO 2011/027897

### SUMMARY OF THE INVENTION

### Problems to be Solved by the Invention

As compared with conventional production methods, the method described in Patent Document 2 is much superior in that it does not require complicated operations and can mass-produce a practical lipidic peptide compound at low cost.

However, a demand has arisen for development of a method for producing a lipidic peptide compound at high yield and high purity suitable for further industrial production.

In view of the aforementioned situations, an object of the present invention is to provide a method capable of producing a lipidic peptide compound at high yield and high purity, as compared with conventional production methods.

### Means for Solving the Problems

In order to achieve the aforementioned object, the present inventors have conducted extensive studies while focusing on a method of removing a non-polar organic solvent before neutralization after completion of the reaction, as well as on raw materials to be used. As a result, the present inventors have found that a lipidic peptide compound can be produced at higher yield and higher purity by a method wherein a non-polar organic solvent is removed by a process involving neutralization of a solution after completion of the reaction by addition of an organic acid to the solution and subsequent phase separation after addition of water and an alcohol to the solution, and a stable ethyl ester is used as a raw material, to thereby reduce the amount of the material added to the system and to allow a homogeneous reaction to occur. The present invention has been accomplished on the basis of this finding.

Accordingly, a first aspect of the present invention is a production method for a lipidic peptide compound of Formula (3) or a pharmaceutically usable salt thereof, the production method comprising:
a reaction step of reacting an ester compound of the following Formula (1): 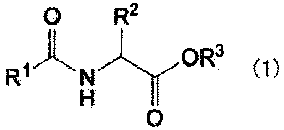 (wherein R¹ is a C₉₋₂₃ aliphatic group having a linear or branched structure; R² is a hydrogen atom or a C₁₋₄ alkyl group possibly having a branched chain having a carbon atom number of 1 or 2; and R³ is a C₁₋₆ alkyl group, a C₁₋₆ haloalkyl group, a C₁₋₆ hydroxyalkyl group, or an aryl group substitutable with a C₁₋₆ alkyl group) with an α-amino acid compound of the following Formula (2): 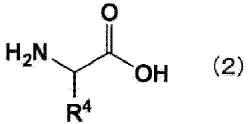 (wherein R⁴ is a -(CH₂)ₙ-X group; n is a number of 1 to 4; and X is an amino group, a guanidino group, a -CONH₂ group, or a 5-membered ring or a 6-membered ring possibly having one to three nitrogen atoms or a fused heterocyclic ring composed of the 5-membered ring and the 6-membered ring) and a base in a solvent containing a non-polar organic solvent;
an extraction step of adding an organic acid to a solution containing a salt of a lipidic peptide prepared through the reaction step and being of the following Formula (3): 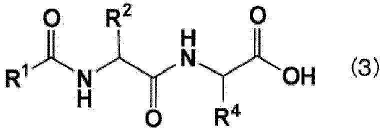 (wherein R¹, R², and R⁴ have the same meanings as defined above) to thereby neutralize the solution, adding water and an alcohol to the neutralized solution, and subjecting the resultant mixture to phase separation, thereby removing the non-polar organic solvent; and
a separation step of removing the lipidic peptide compound of Formula (3) from the solution prepared through the extraction step to the outside of the system.

A second aspect of the present invention is the production method according to the first aspect, characterized in that the solvent contains a non-polar organic solvent and an alcohol.

A third aspect of the present invention is the production method according to the first aspect, wherein, in the aforementioned formula, n is a number of 1 to 4, and X is an amino group, a guanidino group, or a -CONH₂ group; or n is 1, and X is a pyrrole group, an imidazole group, a pyrazole group, or an imidazole group.

A fourth aspect of the present invention is the production method according to the first aspect, wherein, in the aforementioned formula, R¹ is a linear aliphatic group having a carbon atom number of 11 to 21 and possibly having zero to two unsaturated bonds.

A fifth aspect of the present invention is the production method according to the first aspect, wherein, in the aforementioned formula, R² is a hydrogen atom or a C₁₋₃ alkyl group possibly having a branched chain having a carbon atom number of 1.

A sixth aspect of the present invention is the production method according to the first aspect, wherein, in the aforementioned formula, R² is a hydrogen atom, a methyl group, an ethyl group, an n-propyl group, an isopropyl group, an n-butyl group, an isobutyl group, a sec-butyl group, or a tert-butyl group, and R⁴ is an aminomethyl group, an aminoethyl group, a 3-aminopropyl group, a 4-aminobutyl group, a carbamoylmethyl group, a 2-carbamoylethyl group, a 3-carbamoylbutyl group, a 2-guanidinoethyl group, a 3-guanidinopropyl group, a pyrrolemethyl group, an imidazolemethyl group, a pyrazolemethyl group, or a 3-indolemethyl group.

A seventh aspect of the present invention is the production method according to the sixth aspect, wherein, in the aforementioned formula, R² is a hydrogen atom, a methyl group, an isopropyl group, an isobutyl group, or a sec-butyl group, and R⁴ is a 4-aminobutyl group, a carbamoylmethyl group, a 2-carbamoylethyl group, a 3-guanidinopropyl group, an imidazolemethyl group, or a 3-indolemethyl group.

An eighth aspect of the present invention is the production method according to the first aspect, wherein the organic acid is acetic acid.

A ninth aspect of the present invention is the production method according to any one of the first to eighth aspects, wherein the base is at least one selected from among an alkali metal, an alkali metal inorganic acid salt, an alkali metal hydroxide, an alkali metal alkoxide, an alicyclic amine, or an alcohol solution of any of these, or an alcohol dispersion of any of these.

A tenth aspect of the present invention is the production method according to the ninth aspect, wherein the base is at least one selected from among metallic sodium, metallic potassium, sodium carbonate, potassium carbonate, potassium phosphate, sodium phosphate, sodium hydroxide, potassium hydroxide, sodium methoxide, sodium ethoxide, potassium methoxide, potassium ethoxide, t-butoxypotassium, 1,8-diazabicyclo[5.4.0]-7-undecene, 1,5-diazabicyclo[4.3.0]-5-nonene, or an alcohol solution of any of these, or an alcohol dispersion of any of these.

An eleventh aspect of the present invention is the production method according to the tenth aspect, wherein the base is sodium methoxide, or a methanol solution thereof, or a methanol dispersion thereof.

A twelfth aspect of the present invention is the production method according to any one of the first to eleventh aspects, wherein the non-polar organic solvent is at least one selected from the group consisting of an aromatic compound, a saturated aliphatic compound, and an unsaturated aliphatic compound.

A thirteenth aspect of the present invention is the production method according to the twelfth aspect, wherein the non-polar organic solvent is at least one selected from the group consisting of toluene, xylene, o-dichlorobenzene, pentane, hexane, heptane, octane, cyclopentane, cyclohexane, methylcyclohexane, cycloheptane, and 1-hexene.

A fourteenth aspect of the present invention is the production method according to the second aspect, wherein the solvent contains toluene and methanol or ethanol.

A fifteenth aspect of the present invention is the production method according to any one of the first to fourteenth aspects, wherein the reaction between the ester compound of Formula (1) and the α-amino acid compound of Formula (2) is performed at a reaction temperature of 65°C to 75°C.

A sixteenth aspect of the present invention is a production method for a lipidic peptide compound of Formula (3), the production method comprising:
a reaction step of reacting an ester compound of the following Formula (1): 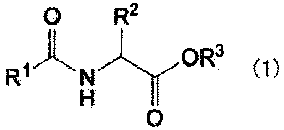 (wherein R¹ is a C₉₋₂₃ aliphatic group having a linear or branched structure; R² is a hydrogen atom or a C₁₋₄ alkyl group possibly having a branched chain having a carbon atom number of 1 or 2; and R³ is a C₁₋₆ alkyl group, a C₁₋₆ haloalkyl group, a C₁₋₆ hydroxyalkyl group, or an aryl group substitutable with a C₁₋₆ alkyl group) with an α-amino acid compound of the following Formula (2): 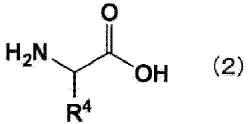 (wherein R⁴ is a -(CH₂)ₙ-X group; n is a number of 1 to 4; and X is an amino group, a guanidino group, a -CONH₂ group, or a 5-membered ring or a 6-membered ring possibly having one to three nitrogen atoms or a fused heterocyclic ring composed of the 5-membered ring and the 6-membered ring) and a base in a solvent containing a non-polar organic solvent;
an extraction step of adding an organic acid to a solution containing a salt of a lipidic peptide prepared through the reaction step and being of the following Formula (3): 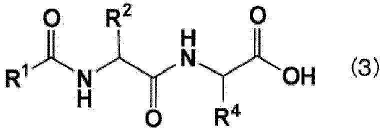 (wherein R¹, R², and R⁴ have the same meanings as defined above) to thereby neutralize the solution, adding water and an alcohol to the neutralized solution, and subjecting the resultant mixture to phase separation, thereby removing the non-polar organic solvent;
a pH adjustment step of adjusting the pH of the solution prepared through the extraction step with a hydrogen halide; and
a separation step of removing the lipidic peptide compound of Formula (3) from the solution prepared through the pH adjustment step to the outside of the system.

A seventeenth aspect of the present invention is a production method for a lipidic peptide compound of Formula (3) or a pharmaceutically usable salt thereof, the production method comprising:
a generation step of reacting a compound of the following Formula (4): 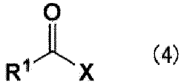 (wherein X is a halogen atom, a C₁₋₆ alkoxy group, or a -OC(O)R¹ group; and R¹ is a C₉₋₂₃ aliphatic group having a linear or branched structure) with a compound of the following Formula (5): 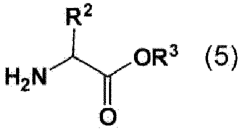 (wherein R² is a hydrogen atom or a C₁₋₄ alkyl group possibly having a branched chain having a carbon atom number of 1 or 2; and R³ is a C₁₋₆ alkyl group, a C₁₋₆ haloalkyl group, a C₁₋₆ hydroxyalkyl group, or an aryl group substitutable with a C₁₋₆ alkyl group), thereby generating an ester compound of the following Formula (1): 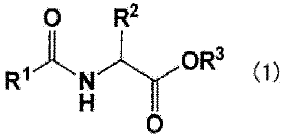 (wherein R¹, R², and R³ have the same meanings as defined above);
a reaction step of reacting the ester compound of Formula (1) generated through the generation step with an α-amino acid compound of the following Formula (2): 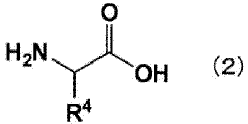 (wherein R⁴ is a hydrogen atom, a C₁₋₇ alkyl group possibly having a branched chain having a carbon atom number of 1 to 3, a phenylmethyl group, a phenylethyl group, or a -(CH₂)ₙ-X group; n is a number of 1 to 4; and X is an amino group, a guanidino group, a -CONH₂ group, or a 5-membered ring or a 6-membered ring possibly having one to three nitrogen atoms or a fused heterocyclic ring composed of the 5-membered ring and the 6-membered ring) and a base in a solvent containing a non-polar organic solvent;
an extraction step of adding an organic acid to a solution containing a salt of a lipidic peptide prepared through the reaction step and being of the following Formula (3): 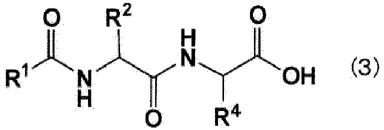 (wherein R¹, R², and R⁴ have the same meanings as defined above) to thereby neutralize the solution, adding water and an alcohol to the neutralized solution, and subjecting the resultant mixture to phase separation, thereby removing the non-polar organic solvent; and
a separation step of removing the lipidic peptide compound of Formula (3) from the solution prepared through the extraction step to the outside of the system.

An eighteenth aspect of the present invention is the production method according to the seventeenth aspect, wherein the reaction between the compound of Formula (4) and the compound of Formula (5) is performed at a reaction temperature of 35°C to 45°C in a homogeneous phase.

### Effects of the present invention

The production method of the present invention can produce a desired lipidic peptide compound and a salt thereof at high purity and high yield, and thus is suitable for industrial-scale production of the compound and salt thereof.

### MODES FOR CARRYING OUT THE INVENTION

In the present specification, "n" denotes normal; "i" denotes iso; "s" or "sec" denotes secondary; "t" or "tert" denotes tertiary; "c" denotes cyclo; "o" denotes ortho; "m" denotes meta; "p" denotes para; "Me" denotes a methyl group; "Bu" denotes a butyl group; and "tBu" denotes a tertiary butyl group.

In Formula (1), R¹ is a C₉₋₂₃ aliphatic group. Preferably, R¹ is a linear aliphatic group having a carbon atom number of 11 to 21, or a linear aliphatic group having a carbon atom number of 11 to 21 and having one to two unsaturated bonds.

Particularly preferred specific examples of the aliphatic group of R¹ include a nonyl group, a decyl group, an undecyl group, a dodecyl group (lauryl group), a tridecyl group, a tetradecyl group (myristyl group), a pentadecyl group, a hexadecyl group (palmityl group), a heptadecyl group (margaryl group), a octadecyl group (stearyl group), a nonadecyl group, an icosyl group, and a henicosyl group.

In Formula (1), R² is a hydrogen atom or a C₁₋₄ alkyl group possibly having a branched chain having a carbon atom number of 1 or 2.

The "C₁₋₄ alkyl group possibly having a branched chain having a carbon atom number of 1 or 2" of R² refers to an alkyl group having a main chain having a carbon atom number of 1 to 4 and possibly having a branched chain having a carbon atom number of 1 or 2. Specific examples of the alkyl group include a methyl group, an ethyl group, an n-propyl group, an i-propyl group, an n-butyl group, an i-butyl group, a sec-butyl group, or a tert-butyl group.

R² is preferably a hydrogen atom or a C₁₋₃ alkyl group possibly having a branched chain having a carbon atom number of 1, more preferably a hydrogen atom. The "C₁₋₃ alkyl group possibly having a branched chain having a carbon atom number of 1" refers to an alkyl group having a main chain having a carbon atom number of 1 to 3 and possibly having a branched chain having a carbon atom number of 1. Specific examples of the alkyl group include a methyl group, an ethyl group, an n-propyl group, an i-propyl group, an i-butyl group, or a sec-butyl group. Preferred is a methyl group, an i-propyl group, an i-butyl group, or a sec-butyl group.

In Formula (1), R³ is a C₁₋₆ alkyl group, a C₁₋₆ haloalkyl group, a C₁₋₆ hydroxyalkyl group, or an aryl group substitutable with a C₁₋₆ alkyl group. Preferred is a C₁₋₆ alkyl group, and more preferred is an ethyl group.

In Formula (2), R⁴ is a -(CH₂)ₙ-X group.

In the -(CH₂)ₙ-X group, n is a number of 1 to 4, and X is an amino group, a guanidino group, a -CONH₂ group, or a 5-membered ring or a 6-membered ring possibly having one to three nitrogen atoms or a fused heterocyclic ring composed of the 5-membered ring and the 6-membered ring.

In the -(CH₂)ₙ-X group, X is preferably an amino group, a guanidino group, a -CONH₂ group, a pyrrole group, an imidazole group, a pyrazole group, or an indole group, more preferably an imidazole group. In the -(CH₂)ₙ-X group, n is preferably 1 or 2, more preferably 1.

Thus, the -(CH₂)ₙ-X group is preferably an aminomethyl group, a 2-aminoethyl group, a 3-aminopropyl group, a 4-aminobutyl group, a carbamoylmethyl group, a 2-carbamoylethyl group, a 3-carbamoylbutyl group, a 2-guanidinoethyl group, a 3-guanidinopropyl group, a pyrrolemethyl group, an imidazolemethyl group, a pyrazolemethyl group, or a 3-indolemethyl group, more preferably a 4-aminobutyl group, a carbamoylmethyl group, a 2-carbamoylethyl group, a 3-guanidinopropyl group, an imidazolemethyl group, or a 3-indolemethyl group, still more preferably an imidazolemethyl group.

Therefore, specific examples of particularly suitable lipidic peptide compounds of Formula (3) are the following compounds each being formed of a lipidic moiety and a dipeptide moiety. The amino acid abbreviations used are histidine (His), glycine (Gly), valine (Val), isoleucine (Ile), alanine (Ala), arginine (Arg), asparagine (Asn), glutamine (Gln), leucine (Leu), lysine (Lys), and tryptophan (Trp). Specific examples of the compounds include N-lauroyl-Gly-His, N-lauroyl-Gly-Trp, N-lauroyl-Gly-Gln, N-lauroyl-Gly-Asn, N-lauroyl-Gly-Arg, N-lauroyl-Gly-Lys, N-lauroyl-Ala-His, N-lauroyl-Ala-Trp, N-lauroyl-Ala-Gln, N-lauroyl-Ala-Asn, N-lauroyl-Ala-Arg, N-lauroyl-Ala-Lys, N-lauroyl-Val-His, N-lauroyl-Val-Trp, N-lauroyl-Val-Gln, N-lauroyl-Val-Asn, N-lauroyl-Val-Arg, N-lauroyl-Val-Lys, N-lauroyl-Leu-His, N-lauroyl-Leu-Trp, N-lauroyl-Leu-Gln, N-lauroyl-Leu-Asn, N-lauroyl-Leu-Arg, N-lauroyl-Leu-Lys, N-lauroyl-Ile-His, N-lauroyl-Ile-Trp, N-lauroyl-Ile-Gln, N-lauroyl-Ile-Asn, N-lauroyl-Ile-Arg, N-lauroyl-Ile-Lys, N-myristoyl-Gly-His, N-myristoyl-Gly-Trp, N-myristoyl-Gly-Gln, N-myristoyl-Gly-Asn, N-myristoyl-Gly-Arg, N-myristoyl-Gly-Lys, N-myristoyl-Ala-His, N-myristoyl-Ala-Trp, N-myristoyl-Ala-Gln, N-myristoyl-Ala-Asn, N-myristoyl-Ala-Arg, N-myristoyl-Ala-Lys, N-myristoyl-Val-His, N-myristoyl-Val-Trp, N-myristoyl-Val-Gln, N-myristoyl-Val-Asn, N-myristoyl-Val-Arg, N-myristoyl-Val-Lys, N-myristoyl-Leu-His, N-myristoyl-Leu-Trp, N-myristoyl-Leu-Gln, N-myristoyl-Leu-Asn, N-myristoyl-Leu-Arg, N-myristoyl-Leu-Lys, N-myristoyl-Ile-His, N-myristoyl-Ile-Trp, N-myristoyl-Ile-Gln, N-myristoyl-Ile-Asn, N-myristoyl-Ile-Arg, N-myristoyl-Ile-Lys, N-palmitoyl-Gly-His, N-palmitoyl-Gly-Trp, N-palmitoyl-Gly-Gln, N-palmitoyl-Gly-Asn, N-palmitoyl-Gly-Arg, N-palmitoyl-Gly-Lys, N-palmitoyl-Ala-His, N-palmitoyl-Ala-Trp, N-palmitoyl-Ala-Gln, N-palmitoyl-Ala-Asn, N-palmitoyl-Ala-Arg, N-palmitoyl-Ala-Lys, N-palmitoyl-Val-His, N-palmitoyl-Val-Trp, N-palmitoyl-Val-Gln, N-palmitoyl-Val-Asn, N-palmitoyl-Val-Arg, N-palmitoyl-Val-Lys, N-palmitoyl-Leu-His, N-palmitoyl-Leu-Trp, N-palmitoyl-Leu-Gln, N-palmitoyl-Leu-Asn, N-palmitoyl-Leu-Arg, N-palmitoyl-Leu-Lys, N-palmitoyl-Ile-His, N-palmitoyl-Ile-Trp, N-palmitoyl-Ile-Gln, N-palmitoyl-Ile-Asn, N-palmitoyl-Ile-Arg, N-palmitoyl-Ile-Lys, N-margaroyl-Gly-His, N-margaroyl-Gly-Trp, N-margaroyl-Gly-Gln, N-margaroyl-Gly-Asn, N-margaroyl-Gly-Arg, N-margaroyl-Gly-Lys, N-margaroyl-Ala-His, N-margaroyl-Ala-Trp, N-margaroyl-Ala-Gln, N-margaroyl-Ala-Asn, N-margaroyl-Ala-Arg, N-margaroyl-Ala-Lys, N-margaroyl-Val-His, N-margaroyl-Val-Trp, N-margaroyl-Val-Gln, N-margaroyl-Val-Asn, N-margaroyl-Val-Arg, N-margaroyl-Val-Lys, N-margaroyl-Leu-His, N-margaroyl-Leu-Trp, N-margaroyl-Leu-Gln, N-margaroyl-Leu-Asn, N-margaroyl-Leu-Arg, N-margaroyl-Leu-Lys, N-margaroyl-Ile-His, N-margaroyl-Ile-Trp, N-margaroyl-Ile-Gln, N-margaroyl-Ile-Asn, N-margaroyl-Ile-Arg, N-margaroyl-Ile-Lys, N-stearoyl-Gly-His, N-stearoyl-Gly-Trp, N-stearoyl-Gly-Gln, N-stearoyl-Gly-Asn, N-stearoyl-Gly-Arg, N-stearoyl-Gly-Lys, N-stearoyl-Ala-His, N-stearoyl-Ala-Trp, N-stearoyl-Ala-Gln, N-stearoyl-Ala-Asn, N-stearoyl-Ala-Arg, N-stearoyl-Ala-Lys, N-stearoyl-Val-His, N-stearoyl-Val-Trp, N-stearoyl-Val-Gln, N-stearoyl-Val-Asn, N-stearoyl-Val-Arg, N-stearoyl-Val-Lys, N-stearoyl-Leu-His, N-stearoyl-Leu-Trp, N-stearoyl-Leu-Gln, N-stearoyl-Leu-Asn, N-stearoyl-Leu-Arg, N-stearoyl-Leu-Lys, N-stearoyl-Ile-His, N-stearoyl-Ile-Trp, N-stearoyl-Ile-Gln, N-stearoyl-Ile-Asn, N-stearoyl-Ile-Arg, N-stearoyl-Ile-Lys, N-elaidoyl-Gly-His, N-elaidoyl-Gly-Trp, N-elaidoyl-Gly-Gln, N-elaidoyl-Gly-Asn, N-elaidoyl-Gly-Arg, N-elaidoyl-Gly-Lys, N-elaidoyl-Ala-His, N-elaidoyl-Ala-Trp, N-elaidoyl-Ala-Gln, N-elaidoyl-Ala-Asn, N-elaidoyl-Ala-Arg, N-elaidoyl-Ala-Lys, N-elaidoyl-Val-His, N-elaidoyl-Val-Trp, N-elaidoyl-Val-Gln, N-elaidoyl-Val-Asn, N-elaidoyl-Val-Arg, N-elaidoyl-Val-Lys, N-elaidoyl-Leu-His, N-elaidoyl-Leu-Trp, N-elaidoyl-Leu-Gln, N-elaidoyl-Leu-Asn, N-elaidoyl-Leu-Arg, N-elaidoyl-Leu-Lys, N-elaidoyl-Ile-His, N-elaidoyl-Ile-Trp, N-elaidoyl-Ile-Gln, N-elaidoyl-Ile-Asn, N-elaidoyl-Ile-Arg, N-elaidoyl-Ile-Lys, N-arachidoyl-Gly-His, N-arachidoyl-Gly-Trp, N-arachidoyl-Gly-Gln, N-arachidoyl-Gly-Asn, N-arachidoyl-Gly-Arg, N-arachidoyl-Gly-Lys, N-arachidoyl-Ala-His, N-arachidoyl-Ala-Trp, N-arachidoyl-Ala-Gln, N-arachidoyl-Ala-Asn, N-arachidoyl-Ala-Arg, N-arachidoyl-Ala-Lys, N-arachidoyl-Val-His, N-arachidoyl-Val-Trp, N-arachidoyl-Val-Gln, N-arachidoyl-Val-Asn, N-arachidoyl-Val-Arg, N-arachidoyl-Val-Lys, N-arachidoyl-Leu-His, N-arachidoyl-Leu-Trp, N-arachidoyl-Leu-Gln, N-arachidoyl-Leu-Asn, N-arachidoyl-Leu-Arg, N-arachidoyl-Leu-Lys, N-arachidoyl-Ile-His, N-arachidoyl-Ile-Trp, N-arachidoyl-Ile-Gln, N-arachidoyl-Ile-Asn, N-arachidoyl-Ile-Arg, N-arachidoyl-Ile-Lys, N-behenoyl-Gly-His, N-behenoyl-Gly-Trp, N-behenoyl-Gly-Gln, N-behenoyl-Gly-Asn, N-behenoyl-Gly-Arg, N-behenoyl-Gly-Lys, N-behenoyl-Ala-His, N-behenoyl-Ala-Trp, N-behenoyl-Ala-Gln, N-behenoyl-Ala-Asn, N-behenoyl-Ala-Arg, N-behenoyl-Ala-Lys, N-behenoyl-Val-His, N-behenoyl-Val-Trp, N-behenoyl-Val-Gln, N-behenoyl-Val-Asn, N-behenoyl-Val-Arg, N-behenoyl-Val-Lys, N-behenoyl-Leu-His, N-behenoyl-Leu-Trp, N-behenoyl-Leu-Gln, N-behenoyl-Leu-Asn, N-behenoyl-Leu-Arg, N-behenoyl-Leu-Lys, N-behenoyl-Ile-His, N-behenoyl-Ile-Trp, N-behenoyl-Ile-Gln, N-behenoyl-Ile-Asn, N-behenoyl-Ile-Arg, and N-behenoyl-Ile-Lys.

Among the aforementioned compounds, more preferred lipidic peptide compounds are N-lauroyl-Gly-His, N-lauroyl-Gly-Trp, N-lauroyl-Gly-Gln, N-lauroyl-Gly-Asn, N-lauroyl-Gly-Lys, N-lauroyl-Ala-His, N-lauroyl-Ala-Trp, N-lauroyl-Ala-Gln, N-lauroyl-Ala-Asn, N-lauroyl-Ala-Lys, N-lauroyl-Val-His, N-lauroyl-Val-Trp, N-lauroyl-Val-Gln, N-lauroyl-Val-Asn, N-lauroyl-Val-Lys, N-myristoyl-Gly-His, N-myristoyl-Gly-Trp, N-myristoyl-Gly-Gln, N-myristoyl-Gly-Asn, N-myristoyl-Gly-Lys, N-myristoyl-Ala-His, N-myristoyl-Ala-Trp, N-myristoyl-Ala-Gln, N-myristoyl-Ala-Asn, N-myristoyl-Ala-Lys, N-myristoyl-Val-His, N-myristoyl-Val-Trp, N-myristoyl-Val-Gln, N-myristoyl-Val-Asn, N-myristoyl-Val-Lys, N-palmitoyl-Gly-His, N-palmitoyl-Gly-Trp, N-palmitoyl-Gly-Gln, N-palmitoyl-Gly-Asn, N-palmitoyl-Gly-Lys, N-palmitoyl-Ala-His, N-palmitoyl-Ala-Trp, N-palmitoyl-Ala-Gln, N-palmitoyl-Ala-Asn, N-palmitoyl-Ala-Lys, N-palmitoyl-Val-His, N-palmitoyl-Val-Trp, N-palmitoyl-Val-Gln, N-palmitoyl-Val-Asn, N-palmitoyl-Val-Lys, N-margaroyl-Gly-His, N-margaroyl-Gly-Trp, N-margaroyl-Gly-Gln, N-margaroyl-Gly-Asn, N-margaroyl-Gly-Lys, N-margaroyl-Ala-His, N-margaroyl-Ala-Trp, N-margaroyl-Ala-Gln, N-margaroyl-Ala-Asn, N-margaroyl-Ala-Lys, N-margaroyl-Val-His, N-margaroyl-Val-Trp, N-margaroyl-Val-Gln, N-margaroyl-Val-Asn, N-margaroyl-Val-Lys, N-stearoyl-Gly-His, N-stearoyl-Gly-Trp, N-stearoyl-Gly-Gln, N-stearoyl-Gly-Asn, N-stearoyl-Gly-Lys, N-stearoyl-Ala-His, N-stearoyl-Ala-Trp, N-stearoyl-Ala-Gln, N-stearoyl-Ala-Asn, N-stearoyl-Ala-Lys, N-stearoyl-Val-His, N-stearoyl-Val-Trp, N-stearoyl-Val-Gln, N-stearoyl-Val-Asn, N-stearoyl-Val-Lys, N-elaidoyl-Gly-His, N-elaidoyl-Gly-Trp, N-elaidoyl-Gly-Gln, N-elaidoyl-Gly-Asn, N-elaidoyl-Gly-Lys, N-elaidoyl-Ala-His, N-elaidoyl-Ala-Trp, N-elaidoyl-Ala-Gln, N-elaidoyl-Ala-Asn, N-elaidoyl-Ala-Lys, N-elaidoyl-Val-His, N-elaidoyl-Val-Trp, N-elaidoyl-Val-Gln, N-elaidoyl-Val-Asn, N-elaidoyl-Val-Lys, N-arachidoyl-Gly-His, N-arachidoyl-Gly-Trp, N-arachidoyl-Gly-Gln, N-arachidoyl-Gly-Asn, N-arachidoyl-Gly-Lys, N-arachidoyl-Ala-His, N-arachidoyl-Ala-Trp, N-arachidoyl-Ala-Gln, N-arachidoyl-Ala-Asn, N-arachidoyl-Ala-Lys, N-arachidoyl-Val-His, N-arachidoyl-Val-Trp, N-arachidoyl-Val-Gln, N-arachidoyl-Val-Asn, N-arachidoyl-Val-Lys, N-behenoyl-Gly-His, N-behenoyl-Gly-Trp, N-behenoyl-Gly-Gln, N-behenoyl-Gly-Asn, N-behenoyl-Gly-Lys, N-behenoyl-Ala-His, N-behenoyl-Ala-Trp, N-behenoyl-Ala-Gln, N-behenoyl-Ala-Asn, N-behenoyl-Ala-Lys, N-behenoyl-Val-His, N-behenoyl-Val-Trp, N-behenoyl-Val-Gln, N-behenoyl-Val-Asn, and N-behenoyl-Val-Lys.

Most preferred compounds are N-lauroyl-Gly-His, N-lauroyl-Gly-Gln, N-lauroyl-Gly-Asn, N-lauroyl-Gly-Lys, N-myristoyl-Gly-His, N-myristoyl-Gly-Gln, N-myristoyl-Gly-Asn, N-myristoyl-Gly-Lys, N-palmitoyl-Gly-His, N-palmitoyl-Gly-Trp, N-palmitoyl-Gly-Gln, N-palmitoyl-Gly-Asn, N-palmitoyl-Gly-Lys, N-palmitoyl-Ala-His, N-palmitoyl-Ala-Trp, N-palmitoyl-Ala-Gln, N-palmitoyl-Ala-Asn, N-palmitoyl-Ala-Lys, N-palmitoyl-Val-His, N-palmitoyl-Val-Trp, N-palmitoyl-Val-Gln, N-palmitoyl-Val-Asn, N-palmitoyl-Val-Lys, N-margaroyl-Gly-His, N-margaroyl-Gly-Gln, N-margaroyl-Gly-Asn, N-margaroyl-Gly-Lys, N-stearoyl-Gly-His, N-stearoyl-Gly-Gln, N-stearoyl-Gly-Asn, N-stearoyl-Gly-Lys, N-elaidoyl-Gly-His, N-elaidoyl-Gly-Gln, N-elaidoyl-Gly-Asn, N-elaidoyl-Gly-Lys, N-arachidoyl-Gly-His, N-arachidoyl-Gly-Gln, N-arachidoyl-Gly-Asn, N-arachidoyl-Gly-Lys, N-behenoyl-Gly-His, N-behenoyl-Gly-Gln, N-behenoyl-Gly-Asn, and N-behenoyl-Gly-Lys.

Examples of the lipidic peptide having a branched structure include N-2-(4,4-dimethylpentan-2-yl)-5,7,7-trimethyloctanoyl-Gly-His and N-2-heptylundecanoyl-Gly-Hi s.

No particular limitation is imposed on the base used for the reaction between the ester compound of Formula (1) and the α-amino acid compound of Formula (2). Examples of the base include alkali metals such as metallic sodium and metallic potassium; alkali metal inorganic acid salts such as sodium carbonate, potassium carbonate, potassium phosphate, and sodium phosphate; alkali metal hydroxides such as sodium hydroxide and potassium hydroxide; alkali metal alkoxides such as sodium methoxide and t-butoxypotassium; aliphatic amines such as triethylamine and tri-n-butylamine; alicyclic amines such as 1,8-diazabicyclo[5.4.0]-7-undecene (hereinafter may be referred to as "DBU") and 1,5-diazabicyclo[4.3.0]-5-nonene (hereinafter may be referred to as "DBN"); aromatic amines such as pyridine and 2-methyl-5-ethylpyridine; and alcohol solutions or alcohol dispersions of these base (solid) compounds. These bases can be used alone or in combination of two or more species.

Among the aforementioned bases, preferred is sodium methoxide, sodium ethoxide, potassium methoxide, potassium ethoxide, t-butoxypotassium, DBU, or DBN, and preferred is sodium methoxide, or an alcohol solution or alcohol dispersion of such a metal alkoxide, in view of increasing percent conversion to thereby further improve the yield of a target product.

Sodium methoxide may be in the form of solid, methanol solution thereof, or methanol dispersion thereof. Alternatively, the sodium methoxide used may be prepared preliminarily or in the reaction system by using metallic sodium and methanol. In consideration of operability and yield, commercially available about 28% sodium methoxide methanol solution is preferably used.

No particular limitation is imposed on the amount of the base used, and the amount is generally about 1 equivalent to 10 equivalents, preferably 1 equivalent to 5 equivalents, more preferably 1.3 equivalents to 2 equivalents, relative to the compound of Formula (1).

No particular limitation is imposed on the non-polar organic solvent contained in the solvent used for the aforementioned reaction, and a non-polar organic solvent that does not affect the reaction may be appropriately selected from various solvents used for general organic synthesis.

Specific examples of the non-polar organic solvent include saturated aliphatic compounds such as pentane, c-pentane, hexane, c-hexane, methyl-c-hexane, heptane, c-heptane, octane, decane, and decalin; unsaturated aliphatic compounds such as 1-hexene and 1-octyne; and aromatic compounds such as benzene, toluene, xylene, and o-dichlorobenzene. These solvents can be used alone or in combination of two or more species.

Among these non-polar organic solvents, preferred is at least one selected from the group consisting of toluene, xylene, o-dichlorobenzene, pentane, hexane, heptane, octane, c-pentane, c-hexane, methyl-c-hexane, c-heptane, and 1-hexene, and particularly preferred is toluene, in view of preventing the hydrolysis of the ester compound of Formula (1), and increasing percent conversion to thereby further improve the yield of a target product.

The solvent used for the aforementioned reaction preferably contains an alcohol in addition to the aforementioned non-polar solvent. No particular limitation is imposed on the alcohol used herein, and an alcohol that does not affect the reaction may be appropriately selected from various alcohol solvents used for general organic synthesis.

Specific examples of the alcohol include methanol, ethanol, n-propanol, i-propanol, n-butanol, i-butanol, s-butanol, t-butanol, n-pentanol, i-pentanol, s-pentanol, t-pentanol, n-hexanol, i-hexanol, s-hexanol, t-hexanol, octanol, decanol, ethylene glycol, 1,3-butanediol, and glycerin. These solvents can be used alone or in combination of two or more species.

The reaction between the ester compound of Formula (1) and the α-amino acid compound of Formula (2) may be performed at any temperature equal to or lower than the boiling point of the solvent used. In consideration of producing a target product within a short period of time at high yield, the reaction temperature is preferably 20°C to 150°C, more preferably 40°C to 80°C, still more preferably 65°C to 75°C.

The reaction time cannot be univocally determined, since it varies with the reaction temperature, and the base and organic solvent species to be used. Generally, the reaction time is about 1 hour to 48 hours.

The reaction may be performed in such a manner that all reagents are mixed at room temperature, and then the mixture is heated to the reaction temperature. Alternatively, the reaction may be controlled by dropwise addition of a necessary reagent. The reaction may be performed in a batch, continuous, reduced-pressure, ambient-pressure, or pressurized manner. More preferably, the reaction is performed in such a manner that a base is added dropwise at ambient pressure.

After completion of the reaction, an organic acid is added to the reaction mixture for neutralization, and then water and an alcohol are added to the mixture, followed by a phase separation operation, to thereby remove the non-polar organic solvent. The organic acid is preferably acetic acid. In consideration of ease of phase separation, the lipidic peptide compound salt is preferably an alkali metal salt.

Thereafter, a hydrogen halide is added to the resultant product preferably in a solvent containing water and an alcohol until the pH of the mixture reaches the previously calculated isoelectric point. For example, after completion of the reaction between the ester compound of Formula (1) and the α-amino acid compound of Formula (2), the non-polar organic solvent is removed, and then a hydrogen halide solution is added to an alcohol-containing aqueous solution of the remaining lipidic peptide compound salt. The isoelectric point, which is also referred to as "isopotential point," is the pH value at which the formal charge becomes zero in the acid-base dissociation state of the corresponding molecule. The isoelectric point value can be calculated from the acid dissociation constant (pKa) of the molecule. For example, the isoelectric point value can be calculated on the basis of the structure of the molecule by using calculation software Calculator Plugins available from ChemAxon. Alternatively, the isoelectric point can be calculated from the actually measured zeta potential value.

The hydrogen halide used in the aforementioned pH adjustment operation is generally in the form of aqueous solution, in view of ease of operation. The hydrogen halide is, for example, hydrochloric acid or hydrobromic acid, and is preferably hydrochloric acid. During the pH adjustment with the hydrogen halide, when the hydrogen halide is used in an amount exceeding the amount required for the pH adjustment, a lipidic peptide hydrochloride is formed, and the recovery rate of a free form is reduced. Thus, care must be taken with the amount of the hydrogen halide used.

After completion of the reaction, an organic acid is added to the resultant solution for neutralization, and then a crude product of the lipidic peptide compound (free form) is recovered by, for example, filtration. If necessary, the crude product is subjected to a post-treatment process, such as washing or recrystallization, to thereby produce a purified product.

The ester compound of Formula (1) used in the present invention can be prepared through reaction between a compound of the following Formula (4) and a compound of the following Formula (5): 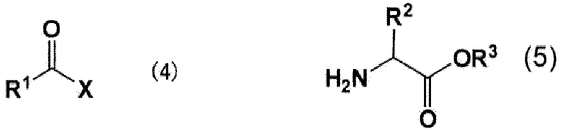 (wherein X, R¹, R², and R³ have the same meanings as defined above).

As described above, the production method of the present invention involves addition of an organic acid to the solution obtained through the reaction for neutralization of the solution, and subsequent addition of water and an alcohol to the resultant mixture, followed by a phase separation operation for removal of the non-polar organic solvent. Thus, the non-polar organic solvent is readily recovered and discarded.

When the lipidic peptide compound has a gelation ability, a polar solvent (e.g., DMF) that has conventionally been used for the production of the lipidic peptide tends to cause gelation after cooling due to the action of the lipidic peptide. In this regard, the production method of the present invention is very useful in the production of the lipidic peptide, since the use of a non-polar organic solvent can prevent gelation.

Further, although the solution obtained through the aforementioned reaction exhibits alkalinity, the use of an aqueous hydrogen chloride solution in an amount required for neutralization of the solution enables completion of the neutralization without causing gelation, and also enables recovery of a free form. A crude crystal of the precipitated free form can be purified by any known technique such as recrystallization, to thereby produce a pure target product.

In the case where the neutralization is not performed, when a phase of an alcohol solution of the lipidic peptide compound salt (i.e., lower phase) is added dropwise to an organic solvent, the lipidic peptide compound salt can be reprecipitated and recovered in the form of solid.

### Examples

The present invention will next be described in more detail with reference to Synthesis Examples, Examples, and Comparative Examples, but the present invention should not be construed as being limited to the following Examples.

The commercially available reagents described below were used in Synthesis Examples and Examples, and the apparatuses described below were used for analysis and measurement of the properties of synthesized compounds.
Methanol: available from KANTO CHEMICAL CO., INC. (Guaranteed Reagent)
Tetrahydrofuran: available from KANTO CHEMICAL CO., INC. (Extra Pure)
i-Propanol: available from KANTO CHEMICAL CO., INC. (Extra Pure)
Toluene: available from KANTO CHEMICAL CO., INC. (Extra Pure)
Acetic acid: available from KANTO CHEMICAL CO., INC. (Extra Pure)
Palmitic acid chloride: available from Aldrich (palmitoyl chloride), NOF CORPORATION (distilled palmitic acid chloride)
Glycine methyl ester hydrochloride: available from Tokyo Chemical Industry Co., Ltd.
Glycine ethyl ester hydrochloride: available from Tokyo Chemical Industry Co., Ltd.
L-Histidine: available from Tokyo Chemical Industry Co., Ltd., KYOWAHAKKO BIO Co., Ltd.
Sodium methoxide 28% methanol solution: available from Nippon Soda Co., Ltd. (liquid sodium methylate 28%), Wako Pure Chemical Industries, Ltd. (28% sodium methoxide methanol solution)
Sodium carbonate: available from JUNSEI CHEMICAL CO., LTD. (first grade), Tokuyama Corporation
Hydrochloric acid: available from KANTO CHEMICAL CO., INC. (first grade)
Acetonitrile: available from KANTO CHEMICAL CO., INC. (special grade)
Amberlyst 15JWET: available from ORGANO CORPORATION
Amberlite FPC3500: available from ORGANO CORPORATION
Kyoward 600: available from Kyowa Chemical Industry Co., Ltd.
GALLEON EARTH V2: available from MIZUSAWA INDUSTRIAL CHEMICALS, LTD.
NMR: JNM-ECP300 (available from JEOL Ltd.)
pH meter: available from Mettler-Toledo
HPLC analysis conditions are as follows.
Column: Inertsil ODS-3 (available from GL sciences)
Developing solvent: MeOH/phosphate buffer (pH = 2.1) = 85/15 (ratio by volume)
* Preparation method for phosphate buffer (pH = 2.1)
Water is added to 7.8 g (50 mmol) of sodium dihydrogenphosphate (NaH₂PO₄2H₂O) and 3.4 mL (50 mmol) of 85% phosphoric acid so that the total amount of the mixture is 1 L.
Oven temperature: 40°C
Detection method: UV 205 nm
Flow rate: 2.0 mL/min
Injection amount: 20 µL
Retention time: N-palmitoyl-Gly-His-methyl: 5.0 min, N-palmitoyl-Gly-His: 5.5 min, N-palmitoyl-Gly: 9.3 min, N-palmitoyl-Gly-methyl: 11.2 min

### [Example 1] Synthesis of N-palmitoyl-Gly-Ethyl

A 500-mL four-necked flask was charged with 15.2 g (0.11 mol) of glycine ethyl ester hydrochloride and 50 g of water. Subsequently, 10.6 g (0.10 mol) of sodium carbonate serving as abase, 75 g of water, and 100 g of toluene serving as an organic solvent were added to the flask, and the resultant mixture was stirred. Thereafter, 25.0 g (0.090 mol) of palmitic acid chloride was added dropwise to the mixture at a reaction temperature of 40 to 45°C over one hour, and the resultant mixture was stirred for two hours. Then, 75 g of 10% salt water was added to the mixture, and a phase separation operation was performed at 60°C. Subsequently, 200 g of toluene was added to the resultant organic phase, and the mixture was subjected to azeotropic dehydration, to thereby produce 248.0 g of a toluene solution of N-palmitoyl-Gly-ethyl (yield: 100%).
¹H-NMR (300 MHz, CDCl₃, δ ppm): 5.94 (1H, m), 2.18 (2H, q, J = 7.5 Hz), 4.03 (2H, d, J = 5.4 Hz), 2.24 (2H, t, J = 7.2 Hz), 1.62 (4H, m), 1.31 (25 H, m), 0.88 (3H, t, J = 7.2 Hz)
MS (CI) m/z: 342.10 (M⁺+1)
Melting point: 79.5°C

### [Example 2]

A 1-L four-necked flask was charged with 14.1 g (0.090 mol) of histidine and 62.1 g of toluene, and 16.7 g (0.086 mol) of 28% sodium methoxide methanol solution serving as a base was added dropwise to the flask. After azeotropic dehydration, the toluene solution of N-palmitoyl-Gly-ethyl produced in Example 1 and 12.4 g of methanol were added to the resultant mixture, and the mixture was heated to 70°C. Thereafter, 13.2 g (0.068 mol) of 28% sodium methoxide methanol solution serving as a base was added dropwise to the mixture, and the resultant mixture was stirred at about 70°C for three hours. After completion of the reaction, the reaction mixture was cooled to 55°C, and the pH of the mixture was adjusted to 7 with acetic acid. Subsequently, 155.3 g of water and 62.1 g of 2-propanol were added to the mixture, and the resultant mixture was subjected to phase separation. The lower phase was removed and mixed with 869.8 g of water, and the temperature of the mixture was adjusted to 40°C. The pH of the mixture was adjusted to 4.5 with 35% hydrochloric acid for neutralization, to thereby precipitate a crude crystal of N-palmitoyl-Gly-His free form. The resultant product was cooled and then subjected to filtration, followed by drying under reduced pressure at 80°C, to thereby prepare 36.5 g of the crude crystal.

To the resultant solid were added 900 g of water and 1,800 g of methanol, and the mixture was heated at 60°C for one hour with stirring. Thereafter, the mixture was left to cool to 25°C, and the precipitated solid was collected by filtration. The same operation was repeated again, and then the resultant solid was dried under reduced pressure. Subsequently, 650 g of tetrahydrofuran was added to the dried solid, and the mixture was stirred at 25°C for one hour. Thereafter, the solid was collected by filtration, and 1,300 g of methanol and 650 g of tetrahydrofuran were added to the resultant solid. The solid was dissolved under heating at 60°C and then cooled to 0°C over two hours, and the mixture was stirred all night at 0°C. The precipitated solid was collected by filtration and then dried under reduced pressure, to thereby produce 31.3 g of a white crystal of N-palmitoyl-Gly-His free form (purity: 100%, yield: 91.1%).

### [Comparative Example 1]

After completion of the reaction in Example 2, the reaction mixture was cooled to 60°C, and 12.7 g of an ion-exchange resin (Amberlite FPC3500) was added to the mixture, whereby the pH of the mixture was changed from 11 to 7. The ion-exchange resin was removed by filtration, and the resultant solution was reprecipitated in 135.0 g of acetonitrile, to thereby produce N-palmitoyl-Gly-His free form.

HPLC analysis of the resultant product showed production of a methylated N-palmitoyl-Gly-His compound (area percentage: 0.2%).

### [Comparative Example 2]

The same process as described above was performed, except that the ion-exchange resin used in Comparative Example 1 was changed to 13.3 g of Amberlyst 15JWET, to thereby produce N-palmitoyl-Gly-His free form.

HPLC analysis of the resultant product showed production of a methylated N-palmitoyl-Gly-His compound (area percentage: 5.6%).

### [Comparative Example 3]

The same process as described above was performed, except that the ion-exchange resin used in Example 3 was changed to 10.0 g of a clay mineral (GALLEON EARTH V2). The pH of the mixture was slightly changed from 11 to 10, and the neutralization was not completed.

### [Comparative Example 4]

The same process as described above was performed, except that the ion-exchange resin used in Example 3 was changed to 10.0 g of magnesium silicate (Kyoward 600). The pH of the mixture was slightly changed from 11 to 10, and the neutralization was not completed.

### [Example 3] Synthesis ofN-palmitoyl-Gly-Ethyl

A 500-L reaction vessel was charged with 5.48 kg (39.3 mol) of glycine ethyl ester hydrochloride and 18 kg of water. Subsequently, 3.82 kg (36.0 mol) of sodium carbonate serving as a base, 27 kg of water, and 36 kg of toluene serving as an organic solvent were added to the reaction vessel, and the resultant mixture was stirred. Thereafter, 9.0 kg (32.7 mol) of palmitic acid chloride was added dropwise to the mixture at a reaction temperature of 40 to 45°C over one hour, and the resultant mixture was stirred for two hours. Then, 27 kg of 10% salt water was added to the mixture, and a phase separation operation was performed at 60°C. Subsequently, 59.6 kg of toluene was added to the resultant organic phase, and the mixture was subjected to azeotropic dehydration, to thereby produce 89.5 kg of a toluene solution of N-palmitoyl-Gly-ethyl (yield: 100%).

### [Example 4]

A200-L reaction vessel was charged with 5.08 kg (32.7 mol) of histidine and 22.4 kg of toluene, and 6.00 kg (31.1 mol) of 28% sodium methoxide methanol solution serving as a base was added dropwise to the reaction vessel. To the resultant mixture were added the toluene solution of N-palmitoyl-Gly-ethyl produced in Example 3 and 4.47 kg of methanol, and the mixture was heated to 70°C. Thereafter, 4.74 kg (24.6 mol) of 28% sodium methoxide methanol solution serving as a base was added dropwise to the mixture, and the resultant mixture was stirred at about 70°C for four hours. After completion of the reaction, the reaction mixture was cooled to 55°C, and the pH of the mixture was adjusted to 7.6 with acetic acid. Subsequently, 55.9 kg of water and 22.4 kg of 2-propanol were added to the mixture, and the resultant mixture was subjected to phase separation. The lower phase was removed and mixed with 313.1 kg of water, and the temperature of the mixture was adjusted to 40°C. The pH of the mixture was adjusted to 4.5 with 35% hydrochloric acid for neutralization, to thereby precipitate a crude crystal of N-palmitoyl-Gly-His free form. The resultant product was cooled and then subjected to filtration, followed by drying under reduced pressure at 80°C, to thereby prepare 36.5 kg of a crude crystal.

The recrystallization was performed in a 500-L reaction vessel in the same manner as in Example 2, to thereby produce 12.0 kg of a white crystal of N-palmitoyl-Gly-His free form (purity: 100%, yield: 91.1%).

## Claims

1. A production method for a lipidic peptide compound of Formula (3) or a pharmaceutically usable salt thereof, the production method comprising:
a reaction step of reacting an ester compound of the following Formula (1): 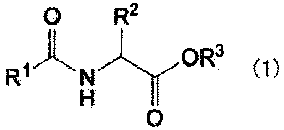
(wherein R¹ is a C₉₋₂₃ aliphatic group having a linear or branched structure; R² is a hydrogen atom or a C₁₋₄ alkyl group possibly having a branched chain having a carbon atom number of 1 or 2; and R³ is a C₁₋₆ alkyl group, a C₁₋₆ haloalkyl group, a C₁₋₆ hydroxyalkyl group, or an aryl group substitutable with a C₁₋₆ alkyl group) with an α-amino acid compound of the following Formula (2): 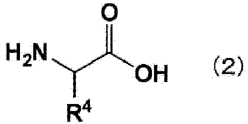
(wherein R⁴ is a -(CH₂)ₙ-X group; n is a number of 1 to 4; and X is an amino group, a guanidino group, a -CONH₂ group, or a 5-membered ring or a 6-membered ring possibly having one to three nitrogen atoms or a fused heterocyclic ring composed of the 5-membered ring and the 6-membered ring) and a base in a solvent containing a non-polar organic solvent;
an extraction step of adding an organic acid to a solution containing a salt of a lipidic peptide prepared through the reaction step and being of the following Formula (3): 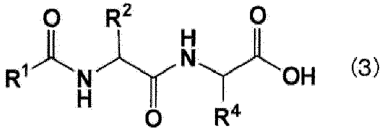 (wherein R¹, R², and R⁴ have the same meanings as defined above) to thereby neutralize the solution, adding water and an alcohol to the neutralized solution, and subjecting the resultant mixture to phase separation, thereby removing the non-polar organic solvent; and
a separation step of removing the lipidic peptide compound of Formula (3) from the solution prepared through the extraction step to the outside of the system.

2. The production method according to claim 1, **characterized in that** the solvent contains a non-polar organic solvent and an alcohol.

3. The production method according to claim 1, wherein, in the aforementioned formula, n is a number of 1 to 4, and X is an amino group, a guanidino group, or a -CONH₂ group; or n is 1, and X is a pyrrole group, an imidazole group, a pyrazole group, or an imidazole group.

4. The production method according to claim 1, wherein, in the aforementioned formula, R¹ is a linear aliphatic group having a carbon atom number of 11 to 21 and possibly having zero to two unsaturated bonds.

5. The production method according to claim 1, wherein, in the aforementioned formula, R² is a hydrogen atom or a C₁₋₃ alkyl group possibly having a branched chain having a carbon atom number of 1.

6. The production method according to claim 1, wherein, in the aforementioned formula, R² is a hydrogen atom, a methyl group, an ethyl group, an n-propyl group, an isopropyl group, an n-butyl group, an isobutyl group, a sec-butyl group, or a tert-butyl group, and R⁴ is an aminomethyl group, an aminoethyl group, a 3-aminopropyl group, a 4-aminobutyl group, a carbamoylmethyl group, a 2-carbamoylethyl group, a 3-carbamoylbutyl group, a 2-guanidinoethyl group, a 3-guanidinopropyl group, a pyrrolemethyl group, an imidazolemethyl group, a pyrazolemethyl group, or a 3-indolemethyl group.

7. The production method according to claim 6, wherein, in the aforementioned formula, R² is a hydrogen atom, a methyl group, an isopropyl group, an isobutyl group, or a sec-butyl group, and R⁴ is a 4-aminobutyl group, a carbamoylmethyl group, a 2-carbamoylethyl group, a 3-guanidinopropyl group, an imidazolemethyl group, or a 3-indolemethyl group.

8. The production method according to claim 1, wherein the organic acid is acetic acid.

9. The production method according to any one of claims 1 to 8, wherein the base is at least one selected from among an alkali metal, an alkali metal inorganic acid salt, an alkali metal hydroxide, an alkali metal alkoxide, an alicyclic amine, or an alcohol solution of any of these, or an alcohol dispersion of any of these.

10. The production method according to claim 9, wherein the base is at least one selected from among metallic sodium, metallic potassium, sodium carbonate, potassium carbonate, potassium phosphate, sodium phosphate, sodium hydroxide, potassium hydroxide, sodium methoxide, sodium ethoxide, potassium methoxide, potassium ethoxide, t-butoxypotassium, 1,8-diazabicyclo[5.4.0]-7-undecene, 1,5-diazabicyclo[4.3.0]-5-nonene, or an alcohol solution of any of these, or an alcohol dispersion of any of these.

11. The production method according to claim 10, wherein the base is sodium methoxide, or a methanol solution thereof, or a methanol dispersion thereof.

12. The production method according to any one of claims 1 to 11, wherein the non-polar organic solvent is at least one selected from the group consisting of an aromatic compound, a saturated aliphatic compound, and an unsaturated aliphatic compound.

13. The production method according to claim 12, wherein the non-polar organic solvent is at least one selected from the group consisting of toluene, xylene, o-dichlorobenzene, pentane, hexane, heptane, octane, cyclopentane, cyclohexane, methylcyclohexane, cycloheptane, and 1-hexene.

14. The production method according to claim 2, wherein the solvent contains toluene and methanol or ethanol.

15. The production method according to any one of claims 1 to 14, wherein the reaction between the ester compound of Formula (1) and the α-amino acid compound of Formula (2) is performed at a reaction temperature of 65°C to 75°C.

16. A production method for a lipidic peptide compound of Formula (3), the production method comprising:
a reaction step of reacting an ester compound of the following Formula (1): 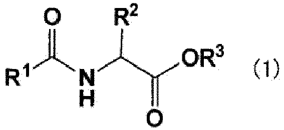
(wherein R¹ is a C₉₋₂₃ aliphatic group having a linear or branched structure; R² is a hydrogen atom or a C₁₋₄ alkyl group possibly having a branched chain having a carbon atom number of 1 or 2; and R³ is a C₁₋₆ alkyl group, a C₁₋₆ haloalkyl group, a C₁₋₆ hydroxyalkyl group, or an aryl group substitutable with a C₁₋₆ alkyl group) with an α-amino acid compound of the following Formula (2): 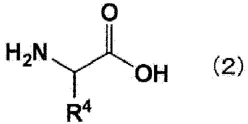
(wherein R⁴ is a -(CH₂)ₙ-X group; n is a number of 1 to 4; and X is an amino group, a guanidino group, a -CONH₂ group, or a 5-membered ring or a 6-membered ring possibly having one to three nitrogen atoms or a fused heterocyclic ring composed of the 5-membered ring and the 6-membered ring) and a base in a solvent containing a non-polar organic solvent;
an extraction step of adding an organic acid to a solution containing a salt of a lipidic peptide prepared through the reaction step and being of the following Formula (3): 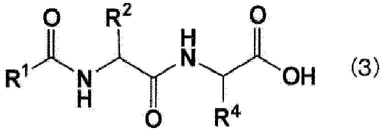 (wherein R¹, R², and R⁴ have the same meanings as defined above) to thereby neutralize the solution, adding water and an alcohol to the neutralized solution, and subjecting the resultant mixture to phase separation, thereby removing the non-polar organic solvent;
a pH adjustment step of adjusting the pH of the solution prepared through the extraction step with a hydrogen halide; and
a separation step of removing the lipidic peptide compound of Formula (3) from the solution prepared through the pH adjustment step to the outside of the system.

17. A production method for a lipidic peptide compound of Formula (3) or a pharmaceutically usable salt thereof, the production method comprising:
a generation step of reacting a compound of the following Formula (4): 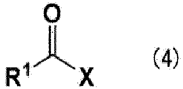
(wherein X is a halogen atom, a C₁₋₆ alkoxy group, or a -OC(O)R¹ group; and R¹ is a C₉₋₂₃ aliphatic group having a linear or branched structure) with a compound of the following Formula (5): 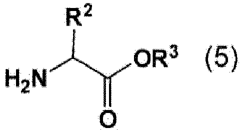
(wherein R² is a hydrogen atom or a C₁₋₄ alkyl group possibly having a branched chain having a carbon atom number of 1 or 2; and R³ is a C₁₋₆ alkyl group, a C₁₋₆ haloalkyl group, a C₁₋₆ hydroxyalkyl group, or an aryl group substitutable with a C₁₋₆ alkyl group), thereby generating an ester compound of the following Formula (1): 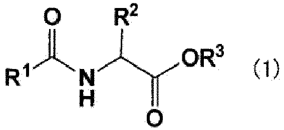
(wherein R¹, R², and R³ have the same meanings as defined above);
a reaction step of reacting the ester compound of Formula (1) generated through the generation step with an α-amino acid compound of the following Formula (2): 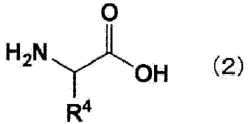 (wherein R⁴ is a hydrogen atom, a C₁₋₇ alkyl group possibly having a branched chain having a carbon atom number of 1 to 3, a phenylmethyl group, a phenylethyl group, or a -(CH₂)ₙ-X group; n is a number of 1 to 4; and X is an amino group, a guanidino group, a -CONH₂ group, or a 5-membered ring or a 6-membered ring possibly having one to three nitrogen atoms or a fused heterocyclic ring composed of the 5-membered ring and the 6-membered ring) and a base in a solvent containing a non-polar organic solvent;
an extraction step of adding an organic acid to a solution containing a salt of a lipidic peptide prepared through the reaction step and being of the following Formula (3): 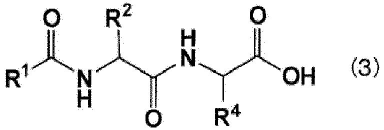 (wherein R¹, R², and R⁴ have the same meanings as defined above) to thereby neutralize the solution, adding water and an alcohol to the neutralized solution, and subjecting the resultant mixture to phase separation, thereby removing the non-polar organic solvent; and
a separation step of removing the lipidic peptide compound of Formula (3) from the solution prepared through the extraction step to the outside of the system.

18. The production method according to claim 17, wherein the reaction between the compound of Formula (4) and the compound of Formula (5) is performed at a reaction temperature of 35°C to 45°C in a homogeneous phase.
